# EUROPEAN PATENT APPLICATION

(11) **EP 4 775 674 A1**
(43) Date of publication of application: **15.07.2026**
(21) Application number: 24845681.6
(22) Date of filing: 25.07.2024
(51) Int. Cl.: C12N 15/09, C12N 5/10, C12N 15/10, C12N 15/63

(54) **GUIDE RNA AND USE THEREOF**

(30) Priority: 26.07.2023 JP 2023121605
(71) Applicant: C4U Corporation, Suita-shi, Osaka, 565-0871 (JP); The University of Tokyo, Bunkyo-ku, Tokyo 113-8654 (JP); RIKEN, Wako-shi, Saitama 351-0198 (JP)
(72) Inventor: MASHIMO, Tomoji, Tokyo 113-8654 (JP); YOSHIMI, Kazuto, Tokyo 113-8654 (JP); TAKESHITA, Kohei, Wako-shi, Saitama 351-0198 (JP); KOBORI, Shungo, Suita-shi, Osaka 565-0871 (JP)
(74) Representative: J A Kemp LLP
(86) International application number: PCT/JP2024/026682
(87) International publication number: WO 2025/023307

(57) **Abstract**

A guide RNA wherein a repeat sequence that is cleaved by processing for forming a cascade complex is arranged on the 5' side of a spacer sequence, and a repeat sequence is not arranged on the 3' side of the spacer sequence, or a sequence that is not cleaved by processing for forming a cascade complex is arranged on the 3' side of the spacer sequence, and a type I CRISPR-Cas system using said guide RNA.

## Description

### [Technical Field]

The present invention relates to a novel form of guide RNA used in a type I CRISPR-Cas system and the use thereof.

### [Background Art]

Genome editing technology is a technology that specifically cleaves a genomic DNA sequence within animal or plant cells and utilizes an intrinsic repair mechanism to freely rewrite the sequence to an arbitrary sequence. Its use is expanding worldwide, not only in bioscience research but also in the breeding of agricultural crops and livestock animals, regenerative medicine, and gene therapy.

The CRISPR-Cas system possessed by bacteria and archaea is divided into Class 1, which cleaves a target sequence with a complex of multiple proteins, and Class 2, which cleaves a target sequence with a single protein. CRISPR-Cas9, CRISPR-Cas12 (Cpf1), and CRISPR-Cas13, which have been developed as genome editing tools to date, are all classified as Class 2.

On the other hand, it was recently found that CRISPR-Cas3, which is a type I CRISPR belonging to Class 1, can be used as a genome editing tool for eukaryotic cells (Patent Literature 1). In this CRISPR-Cas3 system, it has been found that extensive deletion mutations of several hundred to several kb can be introduced with high efficiency around a target sequence in eukaryotic cells such as human cultured cells. Furthermore, compared to CRISPR-Cas9, the target recognition sequence in the guide RNA (crRNA) is longer, and non-specific cleavage is less likely to occur, which is considered to result in high safety.

### [Citation List]

### [Patent Literature]

[PTL 1] International Publication No. WO2018/225858

### [Summary of Invention]

### [Technical Problem]

In the CRISPR-Cas3 system described in the examples of the above-mentioned Patent Literature 1, a pre-crRNA (precursor crRNA) having a full-length repeat sequence at both ends is used, and this has succeeded in causing the processing of the pre-crRNA within eukaryotic cells to form a functional genome editing complex. However, the processing process of the pre-crRNA cannot be said to have been sufficiently studied, and the structure of the guide RNA necessary for the formation of a functional genome editing complex has not been completely elucidated.

The present invention has been made in view of such circumstances, and an object thereof is to clarify the details of the structure of a guide RNA necessary for a type I CRISPR-Cas system to function in eukaryotic cells, and to establish a type I CRISPR-Cas system that utilizes a novel form of guide RNA.

### [Solution to Problem]

As a result of diligent studies to achieve the above object, the present inventors have elucidated that for a type I CRISPR-Cas system to function as a complex, it is necessary for the 5'-side repeat sequence of the guide RNA to undergo cleavage by processing, whereas the processing of the 3'-side repeat sequence is not necessary, and that even when using a repeat sequence after cleavage by processing, a functional complex can be formed.

As a result of further progressing the analysis of this mechanism, the present inventors have found that even when using a truncated crRNA in which the 3'-side repeat sequence is completely deleted, a functional complex is similarly formed, and it is possible to detect nucleic acids and edit DNA.

Furthermore, the present inventor has also found that this mechanism is not limited to a specific subtype but is common to type I CRISPR-Cas systems, leading to the completion of the present invention.

The present invention relates to a guide RNA for a type I CRISPR-Cas system in which the 3' side of a spacer sequence has lost processing sensitivity due to the deletion or modification of the 3'-side repeat sequence, and the use thereof, and more specifically includes the following aspects.
(1) A guide RNA for a type I CRISPR-Cas system, wherein a repeat sequence that is cleaved by processing for forming a cascade complex is arranged on the 5' side of a spacer sequence, and a repeat sequence is not arranged on the 3' side of the spacer sequence, or a sequence that is not cleaved by processing for forming a cascade complex is arranged on the 3' side of the spacer sequence.
(2) A DNA encoding the guide RNA according to (1).
(3) An expression vector comprising the DNA according to (2).
(4) A type I CRISPR-Cas system comprising the guide RNA according to (1).
(5) A method for producing a sample in which a target DNA has been edited, comprising contacting the CRISPR-Cas system according to (4) with a sample comprising the target DNA.
(6) The method according to (5), wherein the sample is a cell.
(7) The method according to (6), wherein the cell is a eukaryotic cell.
(8) A method for detecting a target DNA in a sample, comprising contacting the CRISPR-Cas system according to (4) with the sample.

### [Advantageous Effects of Invention]

According to the present invention, it has been clarified that a type I CRISPR-Cas system can function even if the 3'-side repeat sequence is deleted or modified. As a result, various applications have become possible, such as, for example, the detection of nucleic acids and epigenome editing using a combination of a guide RNA to which an RNA aptamer sequence has been added and an RNA aptamer-recognizing molecule, knock-in using a guide RNA to which a donor sequence has been added, and prime editing using a guide RNA to which a primer binding site sequence and a template sequence for a reverse transcriptase have been added.

### [Brief Description of Drawings]

[Fig. 1] Fig. 1 is a diagram showing the results of an evaluation (CONAN assay) of in vitro cleavage activity by a type I-E CRISPR-Cas system using various forms of crRNA (pre-crRNA, crRNA in which the 5'-side repeat sequence or the 3'-side repeat sequence is of a mature type, and mature crRNA).
[Fig. 2] Fig. 2 is a diagram showing the results of an evaluation (CONAN assay) of in vitro cleavage activity by a type I-E CRISPR-Cas system using a truncated crRNA lacking a 3'-side repeat sequence. As a positive control, a crRNA in which the 3'-side repeat sequence is of a mature type was used.
[Fig. 3] Fig. 3 is a diagram showing the results of an evaluation of genome editing activity by a type I-E CRISPR-Cas system using a truncated crRNA lacking a 3'-side repeat sequence. As a positive control, a pre-crRNA was used.
[Fig. 4] Fig. 4 is a diagram showing the results of an evaluation of genome editing activity by a type I-C CRISPR-Cas system using a truncated crRNA lacking a 3'-side repeat sequence. As a positive control, a pre-crRNA was used.
[Fig. 5] Fig. 5 is a diagram showing the results of an evaluation of genome editing activity by a type I-D CRISPR-Cas system using a truncated crRNA lacking a 3'-side repeat sequence. As a positive control, a pre-crRNA was used.
[Fig. 6] Fig. 6 is a diagram showing an overview of prime editing by a type I-E CRISPR-Cas system using a truncated crRNA lacking a 3'-side repeat sequence, and the results thereof.
[Fig. 7] Fig. 7 is a diagram showing the results of detection of a target DNA by a type I-E CRISPR-Cas system using a truncated crRNA lacking a 3'-side repeat sequence.

### [Description of Embodiments]

### <Guide RNA and Type I CRISPR-Cas System Including the Same>

The present invention provides a novel form of guide RNA in a type I CRISPR-Cas system.

Class 1 CRISPR-Cas systems are classified into type I and type III, and furthermore, type I is classified into six types: type I-A, type I-B, type I-C, type I-D, type I-E, and type I-F, as well as type I-G, which is a subtype of type I-B (see, for example, [van der Oost J et al. (2014) Unravelling the structural and mechanistic basis of CRISPR-Cas systems, Nature Reviews Microbiology, Vol. 12 (No. 7), pp. 479-492], [Jackson RN et al. (2014) Fitting CRISPR-associated Cas3 into the Helicase Family Tree, Current Opinion in Structural Biology, Vol. 24, pp. 106-114]).

In a type I CRISPR-Cas system, a guide RNA forms a complex with a Cas3 protein and cascade proteins, and this complex acts on a target DNA. When using a type I CRISPR-Cas system in a eukaryotic cell, a pre-crRNA (a crRNA in which a repeat sequence that undergoes cleavage by processing is arranged on both sides of a spacer sequence) has typically been used as its guide RNA (the above-mentioned Patent Literature 1), but it was found by the present inventor that cleavage of the repeat sequence on the 3' side of the spacer sequence is not essential in the processing process for forming a cascade complex (hereinafter, sometimes simply referred to as the "processing process").

Therefore, the guide RNA of the present invention is characterized in that a repeat sequence that is cleaved in the processing process is arranged on the 5' side of a spacer sequence, and a repeat sequence is not arranged on the 3' side of the spacer sequence, or a sequence that is not cleaved in the processing process is arranged on the 3' side of the spacer sequence. That is, the guide RNA of the present invention typically has a structure of "repeat sequence that is cleaved in the processing process - spacer sequence" or "repeat sequence that is cleaved in the processing process - spacer sequence - sequence that is not cleaved in the processing process."

The "repeat sequence" in the present invention is a sequence that repeats via a spacer sequence in the CRISPR structure of the bacterial genome from which the type I CRISPR-Cas system is derived.

Although the wild-type repeat sequence differs depending on the subtype of the type I CRISPR-Cas system and the type of bacteria from which it is derived, for example, in a type I-A CRISPR-Cas system derived from Pyrococcus furiosus, it typically consists of the base sequence set forth in SEQ ID NO: 1 having a chain length of 30 bases; in a type I-B CRISPR-Cas system derived from Synechocystis sp., it typically consists of the base sequence set forth in SEQ ID NO: 2 having a chain length of 36 bases; in a type I-C CRISPR-Cas system derived from Neisseria lactamica, it typically consists of the base sequence set forth in SEQ ID NO: 3 having a chain length of 32 bases; in a type I-D CRISPR-Cas system derived from Microcystis aeruginosa, it typically consists of the base sequence set forth in SEQ ID NO: 4 having a chain length of 37 bases; in a type I-E CRISPR-Cas system derived from Escherichia coli, it typically consists of the base sequence set forth in SEQ ID NO: 5 having a chain length of 29 bases; in a type I-F CRISPR-Cas system derived from Pseudomonas aeruginosa, it typically consists of the base sequence set forth in SEQ ID NO: 6 having a chain length of 28 bases; and in a type I-G CRISPR-Cas system derived from Thioalkalivibrio sulfidiphilus, it typically consists of the base sequence set forth in SEQ ID NO: 7 having a chain length of 36 bases.

In the present invention, the repeat sequence on the 5' side of the spacer sequence (hereinafter, sometimes simply referred to as the "5'-side repeat sequence") is typically a wild-type repeat sequence, but a mutation (addition, deletion, substitution, and/or insertion of a base) may be present as long as it is cleaved in the processing process in the same manner as the wild-type repeat sequence. On the other hand, it was found by the present inventor that the cleavage of the repeat sequence on the 3' side of the spacer sequence (hereinafter, sometimes simply referred to as the "3'-side repeat sequence") is not essential in the processing process. Therefore, the guide RNA of the present invention is characterized in that a repeat sequence that is cleaved in the processing process is not arranged on the 3' side of the spacer sequence. On the 3' side of the spacer sequence, a repeat sequence may not be present, and a sequence that is not cleaved in the processing process may be added.

The sequence that is not cleaved in the processing process may be any sequence unrelated to processing, or it may be a repeat sequence that has been modified so that said cleavage does not occur. In a typical processing process, the repeat sequence forms a loop structure, and this loop structure is cleaved by the action of a specific Cas (e.g., Cas6, Cas5, etc.). The cleavage site is typically between the 22nd and 23rd bases of the repeat sequence in type I-A, between the 28th and 29th bases of the repeat sequence in type I-B, between the 19th and 20th bases of the repeat sequence in type I-C, between the 31st and 32nd bases of the repeat sequence in type I-D, between the 21st and 22nd bases of the repeat sequence in type I-E, between the 20th and 21st bases of the repeat sequence in type I-F, and between the 28th and 29th bases of the repeat sequence in type I-G. Therefore, examples of the modified repeat sequence include a repeat sequence that has been truncated so as not to include said cleavage site, and a repeat sequence in which bases around said cleavage site have been modified.

The guide RNA of the present invention is suitable for performing various modifications on the 3' side because cleavage by processing does not occur on the 3' side of the spacer sequence. The modification is not particularly limited, and examples thereof include a label, a tag, a donor sequence, and a sequence for prime editing (a primer binding site sequence and a template sequence for a reverse transcriptase). When using the present invention for prime editing, as shown in the examples of the present application, an RNA aptamer sequence may be further added to the sequence for prime editing. In that case, the reverse transcriptase may be one to which a recognition protein is fused, or one to which a recognition compound is bound. For the RNA aptamer sequence, recognition protein, and recognition compound, refer to the description in <Method for Detecting Target DNA> below.

The "spacer sequence" in the present invention is a sequence designed to be a sequence complementary to a target DNA. The target DNA may be an endogenous DNA or an exogenous DNA. Examples of the endogenous DNA include genomic DNA in chromosomes, mitochondria, and chloroplasts. Examples of the exogenous DNA include reporter genes, marker genes, and genes of viruses, bacteria, protozoa, etc., that infect a host.

The guide RNA of the present invention can be used as an artificially synthesized RNA, or it can be used as an RNA expressed from DNA.

For the expression vector of the guide RNA, various commonly used vectors can be used as a base vector. The type of expression vector is not particularly limited, and a vector capable of expressing the guide RNA in its usage environment (e.g., within a host cell) may be appropriately selected. Examples of the expression vector include plasmid vectors, phage vectors, viral vectors, retroviral vectors, chromosomal vectors, episomal vectors, and vectors derived from viruses (bacterial plasmids, bacteriophages, yeast episomes, etc.), yeast chromosomal elements and viruses (baculovirus, papovavirus, vaccinia virus, adenovirus, fowlpox virus, pseudorabies virus, herpes virus, lentivirus, retrovirus, etc.), and vectors derived from combinations thereof (cosmids, phagemids, etc.). The expression vector may include a promoter sequence for inducing transcription and a sequence for enhancing transcription (e.g., an enhancer sequence).

The expression vector can be prepared by a known method. Examples of such methods include methods described in various manuals, in addition to methods described in instruction manuals attached to kits for vector preparation. For example, [Joseph Sambrook & David W. Russell, Molecular cloning: a laboratory manual 3rd Ed., New York: Cold Spring Harbor Laboratory Press, 2001] is a comprehensive manual.

A Class 1 type I CRISPR-Cas system comprising the guide RNA of the present invention usually includes a Cas3 protein and cascade proteins as constituent elements other than the guide RNA. However, when the purpose is not to cleave the target DNA (e.g., when binding to an expression control region to perform transcriptional repression), it is also possible to use it as a system that does not include a constituent element having nuclease activity (typically, a Cas3 protein, but in type I-D, Cas10d).

Typically, type I-A includes Cas3-HD, Cas3-HEL, Cas5, Cas7, Cas8, and Cas11 as cascade proteins; type I-B includes Cas3, Cas5, Cas6, Cas7, Cas8, and Cas11 as cascade proteins; type I-C includes Cas3, Cas5, Cas7, Cas8, and Cas11 as cascade proteins; type I-D includes Cas3, Cas5, Cas6, Cas7, Cas10, and Cas11 as cascade proteins; type I-E includes Cas3, Cas5, Cas6, Cas7, Cas8, and Cas11 as cascade proteins; type I-F includes Cas2-3, Cas5, Cas6, Cas7, and Cas8 as cascade proteins; and type I-G includes Csb2 (Cas6-like), Cas7, Cas8g, Cas3, and Cas11 as cascade proteins. However, it should be understood that with respect to Cas11, DNA editing activity can be exhibited even when it is excluded from the constituent elements of a type I CRISPR-Cas system (for example, it is known that in type I-B and type I-C, even when Cas11 is not included, DNA editing activity can be exhibited, albeit at a lower level compared to when Cas11 is included, and the present inventor has confirmed that the same is true for type I-D). Therefore, the type I CRISPR-Cas system in the present invention also includes systems that do not include Cas11.

When targeting a chromosomal genome of a eukaryotic cell, it is preferable to add a nuclear localization signal in order to promote the localization of the Cas3 protein and cascade proteins to the nucleus. The nuclear localization signal can be added to the N-terminal side and/or the C-terminal side of each protein. Similarly, for example, when targeting a mitochondrial genome and a chloroplast genome, it is preferable to add a localization signal that promotes localization to them.

In the type I CRISPR-Cas system of the present invention, Cas3 and the cascade may be in the form of proteins, may be in the form of polynucleotides (DNA, RNA) encoding said proteins, or may be in the form of vectors that express said proteins. In the form of a polynucleotide, a modification to a base sequence suitable for expression in a host cell (e.g., codon optimization) may be performed.

In the construction of an expression vector, the DNAs encoding the Cas3 protein and the cascade proteins can be designed to be loaded onto one type of (the same) vector, or can be designed to be loaded all or in part onto separate vectors. In addition, by linking the DNAs encoding each protein via a DNA encoding an amino acid sequence (such as a 2A peptide) that is cleaved by a protease in the cell, it is also possible to express them as a single protein, and then separate them into each protein by the action of the protease for use. The expression vector can be prepared by a known method, as in the case of the above-mentioned guide RNA.

### <Method for Producing Sample in which Target DNA Has Been Edited>

The present invention also provides a method for producing a sample in which a target DNA has been edited, comprising contacting a CRISPR-Cas system comprising the above-mentioned guide RNA with a sample comprising the target DNA.

"DNA editing" in the present invention includes operations consisting of cleavage of DNA at a target site, introduction of a mutation into DNA (deletion, insertion, substitution of a base), modification of a DNA base, expression control of DNA, and combinations thereof. The DNA editing may be performed in vitro or in vivo, depending on the purpose. It may be performed within a cell or in a cell-free system.

One of the main embodiments of DNA editing is the introduction of a mutation via cleavage of a target DNA by a constituent element having nuclease activity in a type I CRISPR-Cas system (typically, a Cas3 protein, but in type I-D, Cas10d) and subsequent DNA repair within the cell. As shown in the examples of the present application, in this embodiment, it is also possible to cause a large-scale deletion in the target DNA.

In another embodiment of DNA editing, depending on the purpose, a Cas3 protein or a cascade protein can be fused with a heterologous protein having a desired activity and used as a chimeric protein. In this embodiment, it is possible to perform various edits on the target DNA according to the activity of the heterologous protein to be fused. Examples of the activity of the heterologous protein to be fused include, but are not limited to, deaminase activity (e.g., cytidine deaminase activity, adenosine deaminase activity), methyltransferase activity, demethylase activity, DNA repair activity, DNA damage activity, dismutase activity, alkylation activity, depurination activity, oxidation activity, pyrimidine dimer formation activity, integrase activity, transposase activity, recombinase activity, polymerase activity (e.g., RNA-dependent DNA polymerase activity when using the present invention for prime editing), ligase activity, photoreactivation enzyme activity, and glycosylase activity. In this case, a mutant in which some or all of the nuclease activity of the Cas3 protein (in the case of type I-D, the Cas10d protein) has been deleted can be used.

Examples of mutants of the Cas3 protein include, for example, in type I-E, a mutant of HD domain H74A (dnCas3), a mutant of SF2 domain motif 1 K320N (dhCas3), and a double mutant of SF2 domain motif 3 S483A/T485A (dh2Cas3). For example, by using a fusion protein of a mutant in which some or all of the nuclease activity of Cas3 has been eliminated and a deaminase as a constituent element of the type I CRISPR-Cas system of the present invention, it becomes possible to perform precise genome editing by substituting a base without causing a deletion at the target site. The method of applying a deaminase to a CRISPR-Cas system is known (Nishida K. et al., Targeted nucleotide editing using hybrid prokaryotic and vertebrate adaptive immune systems, Science, DOI: 10.1126/science.aaf8729, (2016)), and it can be applied to the type I CRISPR-Cas system of the present invention.

By fusing a Cas3 protein or a cascade protein with a desired transcriptional regulatory protein and using it as a chimeric protein, it is possible to regulate the transcription of a gene at a target site. Examples of the transcriptional regulatory protein include, but are not limited to, a photoinducible transcription control factor, a small molecule/drug-responsive transcription control factor, a transcription factor, and a transcription repressor. In this case as well, a mutant in which some or all of the nuclease activity of the Cas3 protein (in the case of type I-D, the Cas10d protein) has been deleted can be used. The method of applying a transcriptional regulatory protein to a CRISPR-Cas system is known to those skilled in the art.

In the present invention, by adding a donor DNA as a constituent element of the type I CRISPR-Cas system, a desired base sequence can be knocked into a target DNA region. The donor DNA usually has homology arms on both sides of the desired base sequence to be knocked in, and the desired base sequence is inserted into the target DNA region via a homologous recombination repair mechanism or the like.

The "sample" used for editing the target DNA is not particularly limited as long as it includes the target DNA. It may be a cell sample (cell, tissue, individual organism) or a cell-free sample.

Cells for editing the target DNA include prokaryotic cells and eukaryotic cells. Prokaryotic cells include bacteria and archaea, and eukaryotic cells include animal cells, plant cells, algal cells, and fungal cells.

Examples of animal cells include mammalian cells, as well as cells of fish, birds, reptiles, amphibians, and insects. Animal cells include, for example, cells constituting an individual animal, cells constituting organs/tissues extracted from an animal, and cultured cells derived from animal tissues. Specifically, examples include germ cells such as oocytes and sperm; embryonic cells at various stages (e.g., one-cell stage embryo, two-cell stage embryo, four-cell stage embryo, eight-cell stage embryo, sixteen-cell stage embryo, morula stage embryo, etc.); stem cells such as induced pluripotent stem (iPS) cells and embryonic stem (ES) cells; and somatic cells such as fibroblasts, hematopoietic cells, neurons, muscle cells, bone cells, hepatocytes, pancreatic cells, brain cells, and kidney cells. As oocytes used for creating genome-edited animals, pre-fertilization and post-fertilization oocytes can be used, but post-fertilization oocytes, that is, fertilized eggs, are preferable. Particularly preferable are fertilized eggs from pronuclear stage embryos. Oocytes that have been cryopreserved can be thawed and used.

Mammals is a concept that includes humans and non-human mammals. Examples of non-human mammals include even-toed ungulates such as cattle, wild boars, pigs, sheep, and goats; odd-toed ungulates such as horses; rodents such as mice, rats, guinea pigs, hamsters, and squirrels; lagomorphs such as rabbits; and carnivores such as dogs, cats, and ferrets. The above-mentioned non-human mammals may be livestock or companion animals, or they may be wild animals.

As a method for producing a non-human individual from a cell, a known method can be used. When producing a non-human individual from a cell in an animal, germ cells or pluripotent stem cells are usually used. For example, molecules constituting the type I CRISPR-Cas system of the present invention are introduced into an oocyte, and the obtained oocyte is then transplanted into the uterus of a female non-human mammal in a pseudopregnant state, after which offspring are obtained. The transplantation can be performed with a fertilized egg at the one-cell stage embryo, two-cell stage embryo, four-cell stage embryo, eight-cell stage embryo, sixteen-cell stage embryo, or morula stage embryo. The oocyte can be cultured under appropriate conditions until transplantation, as necessary. Transplantation and culture of oocytes can be performed based on known methods (Nagy A. et al., Manipulating the Mouse Embryo. Cold Spring Harbor, New York: Cold Spring Harbor Laboratory Press, 2003). From the obtained non-human individual, it is also possible to obtain progeny or clones in which a desired DNA has been edited.

Examples of plant cells include cells of cereals, oil crops, forage crops, fruits, and vegetables. Plant cells include, for example, cells constituting an individual plant, cells constituting organs and tissues isolated from a plant, and cultured cells derived from plant tissues. Examples of plant organs and tissues include leaves, stems, stem apices (growing points), roots, tubers, and calluses. Examples of plants include rice, corn, banana, peanut, sunflower, tomato, rapeseed, tobacco, wheat, barley, potato, soybean, cotton, and carnation, and their propagation materials (e.g., seeds, tuberous roots, tubers, etc.) are also included.

In plants, it has long been known that their somatic cells have totipotency, and methods for regenerating a plant body from a plant cell have been established in various plants. Therefore, for example, by introducing molecules constituting the type I CRISPR-Cas system of the present invention into a plant cell and regenerating a plant body from the obtained plant cell, a plant body in which a desired DNA has been edited can be obtained. From the obtained plant body, it is also possible to obtain progeny, clones, or propagation materials in which a desired DNA has been edited. As a method for obtaining an individual by redifferentiating plant tissue through tissue culture, a method established in this technical field can be used (Transformation Protocol [Plant Edition] edited by Yutaka Tabei, Kagaku-Dojin, pp. 340-347 (2012)).

The method for introducing the type I CRISPR-Cas system of the present invention into a cell is not particularly limited. Examples thereof include methods such as the electroporation method, calcium phosphate method, liposome method, DEAE-dextran method, microinjection method, cationic lipid-mediated transfection, electroporation, transduction, and infection using a viral vector. Such methods are described in many standard laboratory manuals, such as "Leonard G. Davis et al., Basic methods in molecular biology, New York: Elsevier, 1986."

### <Method for Detecting Target DNA>

The present invention also provides a method for detecting a target DNA in a sample, comprising contacting a CRISPR-Cas system comprising the above-mentioned guide RNA with the sample.

As the "sample" in the detection method of the present invention, a desired sample in which a target DNA is to be detected (hereinafter referred to as a "test sample") can be used. The test sample may be a cell sample or a cell-free sample, and examples thereof include a biological tissue, a cell, a cell lysate, a body fluid (urine, saliva, serum, plasma, whole blood, etc.), or a sample including a purified or synthesized DNA.

One embodiment of the detection method of the present invention is a method that utilizes a label added to the guide RNA. Since the guide RNA of the present invention does not undergo cleavage by processing on the 3' side of the spacer sequence, a target DNA can be detected by using a guide RNA to which a label has been added on the 3' side.

The label is not particularly limited as long as it is detectable, but examples thereof include RNA aptamer sequences/recognition proteins such as MS2/MCP, PP7/PCP, boxB/AN22P, and Pepper/tDeg, and RNA aptamer sequences/recognition compounds such as MS2/MCP, PP7/PCP, boxB/AN22P, and Pepper/tDeg.

When using an RNA aptamer sequence/recognition protein, by adding an RNA aptamer sequence to the guide RNA and binding a recognition protein fused with a fluorescent protein such as GFP to said RNA aptamer sequence, the target DNA can be detected using fluorescence as an indicator (Yang LZ et al., Cell Insight 1 (2022) 100044). When using an RNA aptamer sequence/recognition compound, by adding an RNA aptamer sequence to the guide RNA and binding a recognition compound to said RNA aptamer sequence, the target DNA can be detected using color development as an indicator.

In these detections, in order to avoid degradation of the target DNA by the nuclease activity of the type I CRISPR-Cas system, it is preferable to use a system that has lost nuclease activity, for example, a system that utilizes a Cas3 protein that has lost nuclease activity (in type I-D, Cas10d) or a system from which the Cas3 protein (in type I-D, Cas10d) has been excluded.

Another embodiment of the detection method of the present invention is a method that utilizes a single-stranded probe DNA (see Example 1). It is known that in a type I CRISPR-Cas system, when it recognizes and binds to a target DNA in a sample, it indiscriminately cleaves single-stranded DNA (ssDNA) present in the vicinity, and it is also possible to detect the target DNA by utilizing this phenomenon (International Publication No. WO2021/149829).

Specifically, by co-mixing a single-stranded probe DNA whose cleavage is detectable in a reaction system including a sample for detecting a target DNA and a type I CRISPR-Cas system, the target DNA in the sample can be detected using the signal generated by the cleavage of said single-stranded probe DNA as an indicator. When using this detection principle, it is not necessary to label the guide RNA of the present invention itself.

The single-stranded probe DNA is not particularly limited as long as its cleavage is detectable, and it may be linear or circular, but it is preferably linear. The nucleic acid constituting the single-stranded probe DNA may include one or more modifications (e.g., base modification, backbone modification, sugar modification).

One preferred embodiment of the label bound to the single-stranded probe DNA is a fluorophore/quencher pair. In this embodiment, when the fluorophore/quencher pair is bound to the single-stranded probe DNA and is in proximity, the signal from the fluorophore is reduced or eliminated, but when the single-stranded probe DNA is cleaved and the fluorophore is separated from the quencher, a sufficient signal from the fluorophore is detected. Therefore, the target DNA in the test sample can be detected using the fluorescence signal generated from the single-stranded probe DNA cleaved by the type I CRISPR-Cas system that recognized and bound to the target DNA as an indicator.

Another preferred embodiment of the label bound to the single-stranded probe DNA is a donor/acceptor pair for Förster resonance energy transfer (FRET). In this embodiment, when the donor/acceptor pair is bound to the single-stranded probe DNA and is in proximity, excitation of the donor causes excitation and emission of the acceptor (i.e., a FRET signal is generated), but when the single-stranded probe DNA is cleaved and the donor and acceptor are separated, the FRET signal is reduced or eliminated. Therefore, the target DNA in the test sample can be detected using the reduction or elimination of the FRET signal in the single-stranded probe DNA cleaved by the type I CRISPR-Cas system that recognized and bound to the target DNA as an indicator.

In addition, for the detection of the single-stranded probe DNA, for example, immunochromatography (lateral flow assay) can also be used.

The contacting of the test sample, the type I CRISPR-Cas system, and the single-stranded probe DNA can be performed, for example, by mixing them. When the test sample includes cells, it can include further operations for introducing the CRISPR-Cas system and the single-stranded probe DNA into the cells in the test sample.

### [Examples]

Hereinafter, the present invention will be described in more detail by way of examples, but the present invention is not limited to the following examples.

### [Example 1] In Vitro DNA Cleavage Activity at Various crRNA Lengths

In this detection system, a type I-E CRISPR derived from E. coli was used. The Cas3 protein was expressed and purified using insect cells Sf9. Specifically, after integrating a Cas3 gene with a His-tagged nuclear localization signal (bpNLS) into a baculovirus gene expression vector, a baculovirus was created using the E. coli strain DH10bac. After infecting insect cells Sf9 with the created baculovirus to express the Cas3 protein, all proteins were collected, and the Cas3 protein was purified by a nickel column and gel filtration purification.

Cascade components were similarly expressed and purified using insect cells Sf9. Specifically, a baculovirus gene expression vector was created in which the genes for Cas5, Cas6, Cas7, Cas8, and Cas11, each with an added His-tagged nuclear localization signal (bpNLS), were linked by a 2A peptide sequence, the proteins were expressed in the same manner as above, and the Cas3 protein was purified by a nickel column and gel filtration purification.

crRNAs were obtained by synthesizing double-stranded DNA fragments encoding each, followed by in vitro transcription and then column purification. In this experiment, a pre-crRNA (SEQ ID NO: 8), a mature crRNA (SEQ ID NO: 9), a 5'-mature type crRNA (a crRNA in which only the 5'-side repeat sequence is of a mature type), and a 3'-mature type crRNA (a crRNA in which only the 3'-side repeat sequence is of a mature type) were used.

In order to examine the change in the cleavage activity of the CRISPR-Cas3 system due to crRNA length, a measurement (CONAN assay) of non-specific cleavage (collateral cleavage activity) observed upon target recognition was performed using a fluorescence quencher probe (56FAM-AAGGTCGGA-ZEN-GTCAACGGATTTGGTC-ABkFQ, IDT, Inc.; the sequence between ZEN and ABkFQ is set forth in SEQ ID NO: 10).

First, a cascade factor (0.8 µg/µL) and a crRNA (250 ng/uL) were mixed in equal amounts, and incubated for 10 minutes at 37°C in a complex formation buffer (5 mM HEPES-K pH 7.5, 60 mM KCl, 10 mM MgCl₂, 10 µM CoCl₂) to form a cascade complex. Next, in a reaction buffer (5 mM HEPES-K pH 7.5, 60 mM KCl, 10 mM MgCl₂, 10 µM CoCl₂, 1 mM ATP), a Cas3 protein (final concentration 40 ng/µL), a cascade complex (final concentration 32 ng/µL), a double-stranded DNA fragment (final concentration 5 nM) including a target sequence (mouse Tyr gene/SEQ ID NO: 11), and a fluorescence quencher probe (final concentration 1 µM) were mixed and prepared in a 10 µL system. Using a real-time PCR instrument (CFX96 Touch Deep Well system, Bio-Rad Laboratories, Inc.), the intensity of the FAM signal was measured over time every 30 seconds at 37°C.

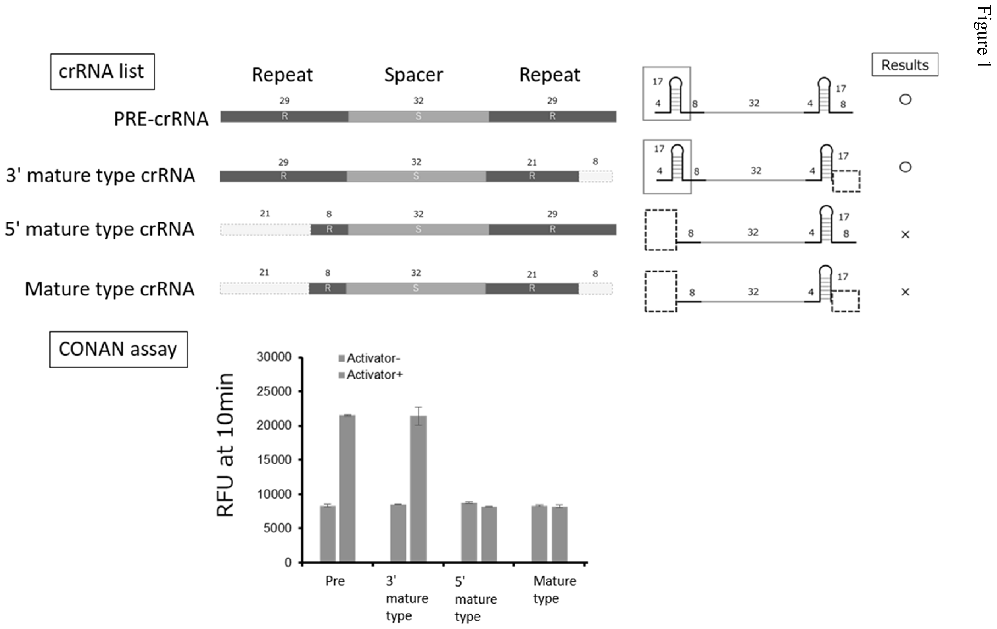

As a result, for the 5'-side repeat sequence, a fluorescence signal due to collateral cleavage activity was detected only when it was in the precursor state, whereas for the 3'-side repeat sequence, a fluorescence signal was detected in both the precursor and mature states (Fig. 1). That is, the 5'-side repeat sequence was essential for the complex formation and activation of CRISPR-Cas3.

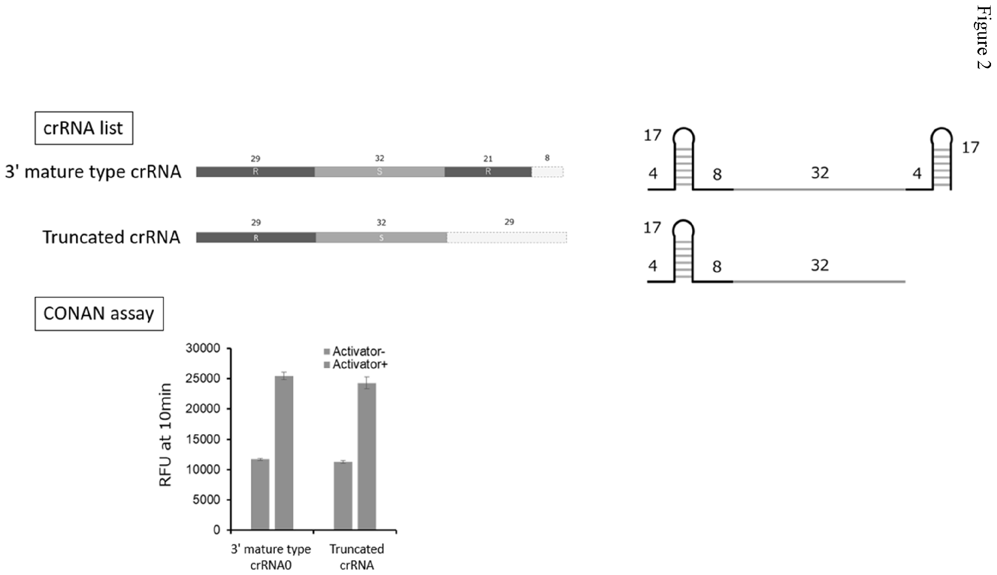

Therefore, next, it was examined whether the 3'-side repeat sequence could be further shortened. As a result, a fluorescence signal due to collateral cleavage activity was detected even in the state of a truncated crRNA (SEQ ID NO: 12) in which the 3'-side repeat sequence was absent (Fig. 2). That is, the 21 bases on the 3' side that remain in the maturation process were not themselves essential for the complex formation and activation of CRISPR-Cas3.

From the above results, it became clear that when the cascade factor and the crRNA form a complex, the 5'-side repeat sequence is essential, whereas the 3'-side repeat sequence is not essential, and that even a truncated crRNA in which the 3'-side repeat sequence is completely lost exhibits activity.

### [Example 2] Measurement of Genome Cleavage Activity in Human Cultured K562 Cells Using Type I-E Truncated crRNA

A purified human EMX1 target crRNA (truncated form; 20 ng/µL) was introduced into K562 cells that doxycycline-inducibly express Cas3 and cascade components (Cas5, Cas6, Cas7, Cas8, Cas11) by an electroporation method using a 4D-Nucleofector System (Lonza Inc.). The cells were placed in a medium supplemented with 2 µg/ml doxycycline, and after culturing for 2 days at 37°C and 5% CO₂, all cells were collected, the genome was extracted, and PCR was performed with a primer set that amplifies 3.8 kb including the PAM region of the EMX gene.

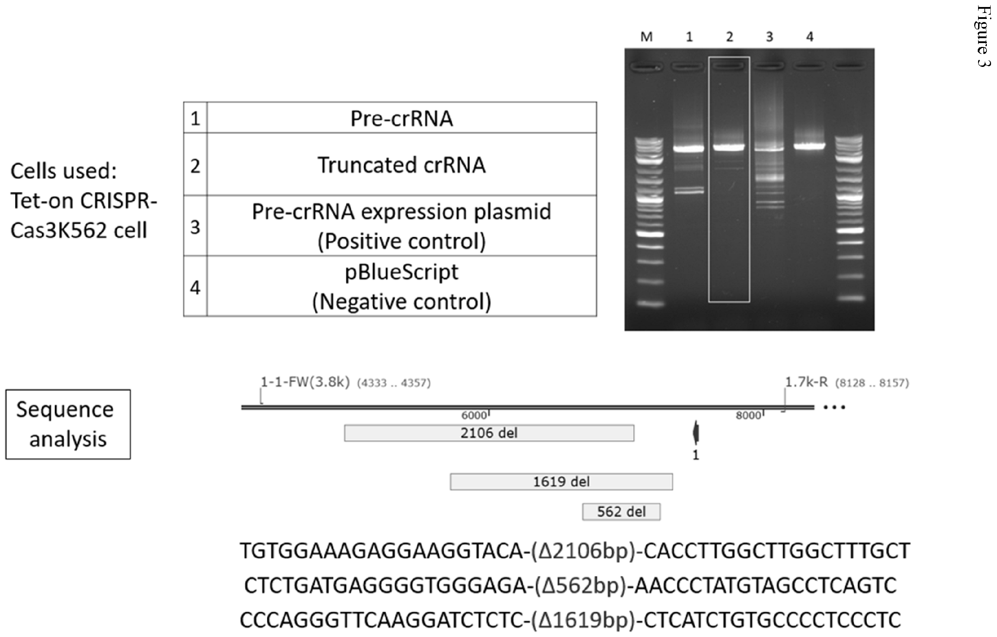

As a result, when the purified truncated crRNA (SEQ ID NO: 14) was used, a shorter band was detected compared to the wild-type band, similar to the purified pre-crRNA (SEQ ID NO: 13) and plasmid-expressed pre-crRNA (Fig. 3).

In order to characterize the genome editing, as a result of performing long-read sequencing analysis on the PCR product using a nanopore sequencer (Oxford Nanopore Technologies plc), deletions of 562 base pairs, 1619 base pairs, and 2106 base pairs were observed upstream of the 5' PAM of the spacer sequence in the target region (Fig. 3). This confirmed that a truncated crRNA can be used for genome editing in human cells.

### [Example 3] Measurement of Genome Cleavage Activity in Human Cultured HEK293T Cells Using Type I-C Truncated crRNA

In order to verify whether a similar phenomenon occurs in other Type I CRISPRs, it was examined whether a truncated crRNA introduces a mutation into the human EMX1 gene using the type I-C system of Neisseria lactamica.

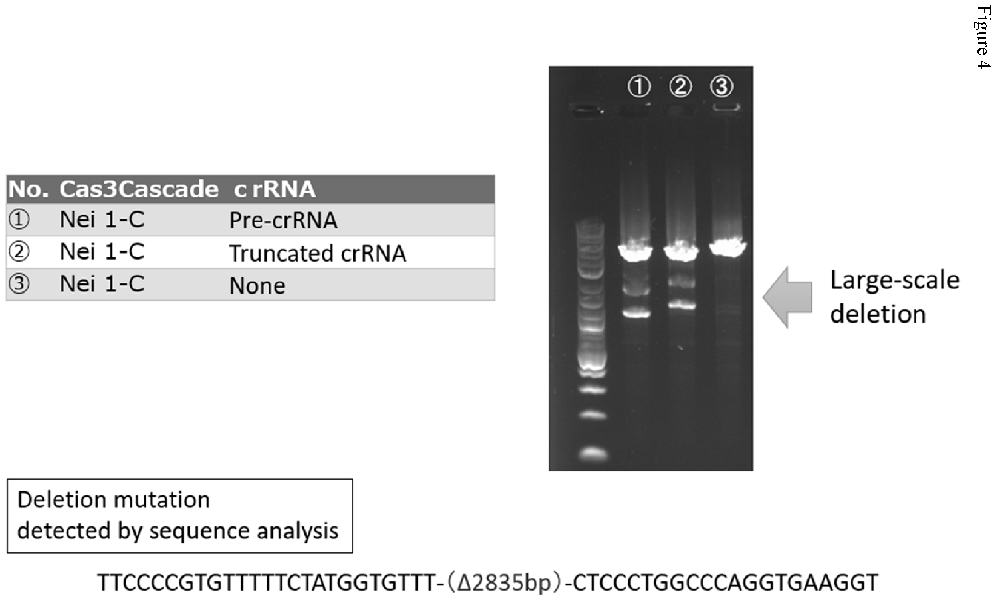

A plasmid expressing a pre-crRNA (SEQ ID NO: 15) or a truncated crRNA (SEQ ID NO: 16) was prepared and introduced into HEK293T cells together with a plasmid expressing five Cas (3, 5, 7, 8, 11) effectors and a puromycin resistance gene, using Lipofectamine 2000 (Thermo Fisher Scientific Inc.). The cells were placed in a medium supplemented with 1 µg/ml puromycin, and after culturing for 2 days at 37°C and 5% CO₂, all cells were collected, the genome was extracted, and PCR was performed using a primer set that amplifies 3.8 kb including the PAM region of the EMX gene. As a result of performing electrophoresis on the PCR product, it was confirmed that the truncated crRNA, similar to the pre-crRNA, forms a shorter band compared to the wild-type band (Fig. 4).

A band with a lower molecular weight than the wild-type band was cut out, purified, and as a result of performing Sanger sequencing analysis, a deletion of 2835 base pairs was observed (Fig. 4). This confirmed that in Type I-C CRISPR as well, a truncated crRNA can be used for genome editing in human cells.

### [Example 4] Measurement of Genome Cleavage Activity in Human Cultured HEK293T Cells Using Type I-D short form crRNA

In order to verify whether a similar phenomenon occurs in other Type I CRISPRs, it was examined whether a truncated crRNA introduces a mutation into the human EMX1 gene using the type I-D system of Microcystis aeruginosa.

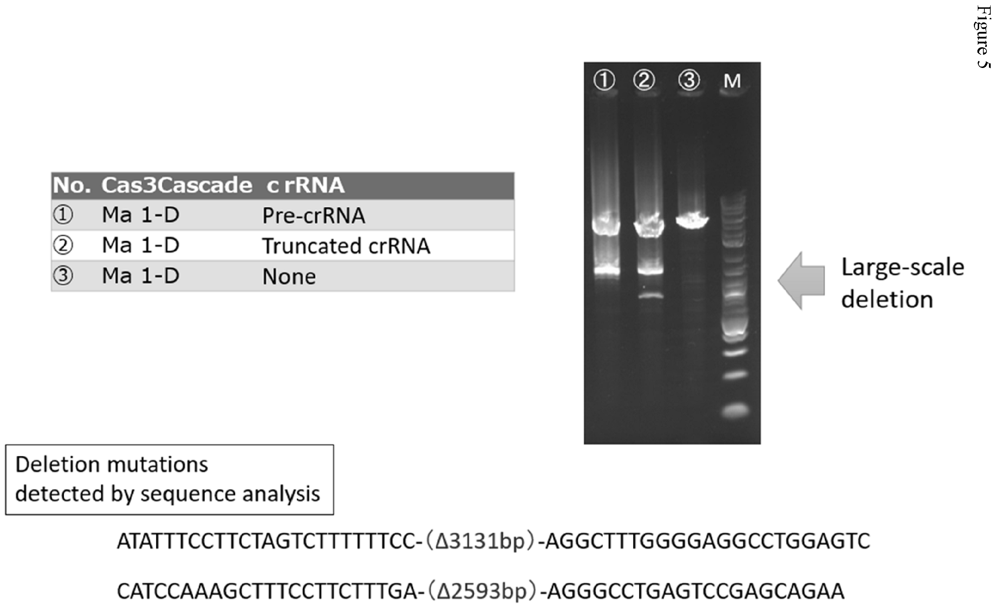

Plasmids expressing a pre-crRNA (SEQ ID NO: 17) and a truncated crRNA (SEQ ID NO: 18), respectively, were prepared and introduced into HEK293T cells together with a plasmid expressing six Cas (3, 5, 6, 7, 10, 11) effectors and a puromycin resistance gene, using Lipofectamine 2000 (Thermo Fisher Scientific Inc.). The cells were placed in a medium supplemented with 1 µg/ml puromycin, and after culturing for 2 days at 37°C and 5% CO₂, all cells were collected, the genome was extracted, and PCR was performed using a primer set that amplifies 3.8 kb including the PAM region of the EMX gene. As a result of electrophoresis of the PCR product, it was confirmed that the truncated crRNA, similar to the pre-crRNA, forms a shorter band compared to the wild-type band (Fig. 5).

A band of the PCR product with a smaller molecular weight than the wild-type band was cut out, purified, and as a result of performing Sanger sequencing analysis, deletions of 3131 base pairs and 2593 base pairs were observed (Fig. 5). This confirmed that in Type I-D CRISPR as well, a truncated crRNA can be used for genome editing in human cells.

### [Example 5] Verification of Prime Editing by Modified crRNA Having Donor Sequence

In a type I-E CRISPR-Cas3 system, it was verified whether a HiBiT sequence could be inserted in a target-specific manner using a truncated crRNA (modified crRNA) to which a 33-base HiBiT sequence and an MS2 sequence were added on the 3' side.

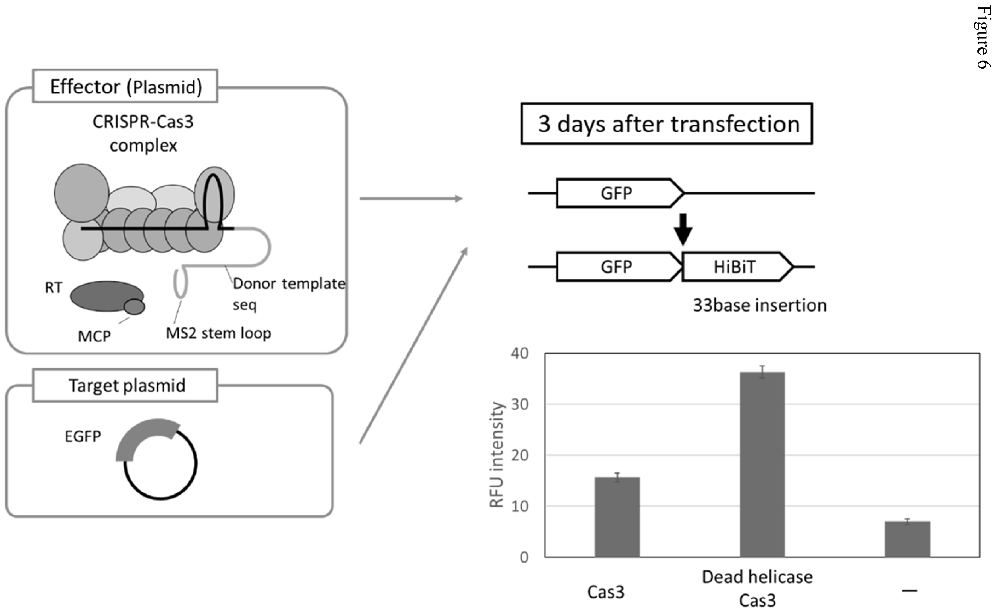

The stop codon site of the GFP gene was selected as the target, and a plasmid encoding a modified pre-crRNA and six Cas (3, 5-8, 11) effectors was prepared. The Cas3 used here was a wild-type or its mutant (a double mutant of S483A/T485A of SF2 domain motif 3 (dh2Cas3)). A plasmid encoding the modified pre-crRNA, etc., a CAG-GFP expression plasmid, and an MCP-Reverse Transcriptase expression plasmid were lipofected into 293T cells, and the cells were cultured and passaged. Three days later, the cells were lysed, and the luciferase activity was measured using a Nano-Glo HiBiT Lytic Detection System (Fig. 6). As a result, an increase in luciferase activity was observed when the helicase-deficient Cas3 was used, which suggested that HiBiT was inserted into the GFP site (graph in the lower right of Fig. 6).

### [Example 6] Verification of GFP Accumulation by Modified crRNA Having MS2 Tandem Sequence

In a type I-E CRISPR-Cas3 system, it was verified whether a truncated crRNA (modified crRNA) to which an MS2 tandem sequence was added on the 3' side could be inserted in a target-specific manner.

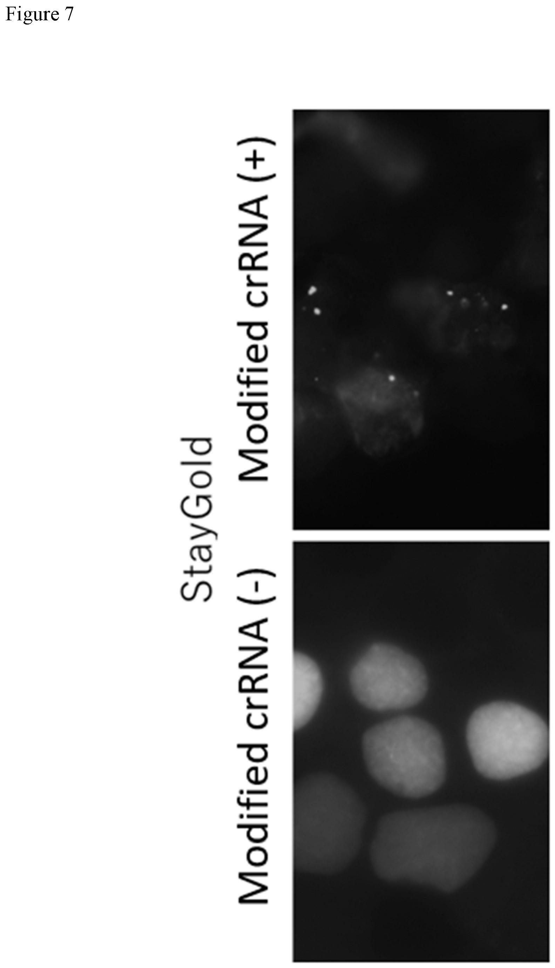

The stop codon site of the GFP gene was selected as the target, and a plasmid encoding a modified pre-crRNA and five Cas (5-8, 11) effectors was prepared. A plasmid encoding the modified pre-crRNA, etc., and an MCP-EGFP (StayGold) expression plasmid were lipofected into 293T cells, and the cells were cultured and passaged. As a result of observing the cells with a fluorescence microscope two days later, an accumulation of GFP was observed when the modified crRNA was used, which suggested that the target sequence can be imaged (Fig. 7).

### [Industrial Applicability]

In a type I CRISPR-Cas system using the truncated crRNA of the present invention, since a guide RNA on which various modifications such as label addition have been performed can be used, it is suitable for use in a wide range of fields, not only for genome editing of animals and plants, but also for diagnosis by nucleic acid detection.

## Claims

1. A guide RNA for a type I CRISPR-Cas system, wherein a repeat sequence that is cleaved in a processing process for forming a cascade complex is arranged on the 5' side of a spacer sequence, and a repeat sequence is not arranged on the 3' side of the spacer sequence, or a sequence that is not cleaved in a processing process for forming a cascade complex is arranged on the 3' side of the spacer sequence.

2. A DNA encoding the guide RNA according to claim 1.

3. An expression vector comprising the DNA according to claim 2.

4. A type I CRISPR-Cas system comprising the guide RNA according to claim 1.

5. A method for producing a sample in which a target DNA has been edited, comprising contacting the CRISPR-Cas system according to claim 4 with a sample comprising the target DNA.

6. The method according to claim 5, wherein the sample is a cell.

7. The method according to claim 6, wherein the cell is a eukaryotic cell.

8. A method for detecting a target DNA in a sample, comprising contacting the CRISPR-Cas system according to claim 4 with the sample.
